# EUROPEAN PATENT APPLICATION

(11) **EP 3 744 228 A1**
(43) Date of publication of application: **02.12.2020**
(21) Application number: 18900734.7
(22) Date of filing: 24.12.2018
(51) Int. Cl.: A61B 3/103, A61B 3/14

(54) **RETINAL DIGITAL IMAGING SYSTEM, RETINAL DIGITAL IMAGING INSTRUMENT, AND RETINAL DIGITAL IMAGING METHOD**

(30) Priority: 22.01.2018 CN 201810063325
(71) Applicant: Shenzhen Thondar Technology Co., Ltd, Shenzhen, Guangdong 518000 (CN)
(72) Inventor: WANG, Ningli, Shenzhen, Guangdong 518000 (CN); HUANG, Yequan, Shenzhen, Guangdong 518000 (CN); ZHANG, Jinsong, Shenzhen, Guangdong 518000 (CN); REN, Jianwei, Shenzhen, Guangdong 518000 (CN); MA, Chao, Shenzhen, Guangdong 518000 (CN); CHEN, Zhenhua, Shenzhen, Guangdong 518000 (CN); DONG, Xuechuan, Shenzhen, Guangdong 518000 (CN); ZHEN, Yi, Shenzhen, Guangdong 518000 (CN); LIU, Kexing, Shenzhen, Guangdong 518000 (CN); RIBARIC, Zeljko, Shenzhen, Guangdong 518000 (CN)
(74) Representative: Osterhoff, Utz
(86) International application number: PCT/CN2018/123241
(87) International publication number: WO 2019/141051

(57) **Abstract**

Retina digital imaging system, instrument and method A retina digital imaging system includes: an illumination module (1), a main optical assembly (3), and an image sensor module (2); the illumination module (1) includes light-emitting diodes capable of emitting light with different wavelengths, and each light-emitting diode emits light to enter the retina through the main optical assembly (3) to form an illumination optical path; the light reflected by the retina passes through the main optical assembly (3) and forms an image on the image sensor module (2) to form an imaging optical path. The various spectral bands formed by light-emitting diodes may form a wider spectrum, so different layers of the retina may be imaged to provide valuable medical and diagnostic data.

## Description

### CROSS REFERENCE TO RELATED DISCLOSURE

The present disclosure claims the priority of Chinese Patent Application with No. 2018100633252, entitled "RETINA DIGITAL IMAGING SYSTEM AND RETINA DIGITAL IMAGER", filed on January 22, 2018, which is hereby incorporated by reference in its entirety.

### FIELD

The present disclosure relates to the technical field of medical optical instruments, and in particular, to a retina digital imaging system, a retina digital imaging instrument, and a retina digital imaging method.

### BACKGROUND

Retina imaging technology is gradually widely used in medical and biometric technologies. In medicine, timely detection and tracking of retinopathy can play an effective role in diagnosis and early warning of various diseases. In the field of biometric recognition, the retina has far more biological features than fingerprints, palm prints, etc., which can greatly improve the recognition accuracy; and the retina is deep into the eye, which is not easy to be obtained by the outside world, and has a very high confidentiality.

Retina imaging technology has a long history of research, but it still cannot meet the needs of society. For example, in the medical field, with the development of the Internet, telemedicine systems have gradually become an indispensable part of medical diagnosis, but conventional retina lighting currently uses halogen or white light for retina illumination, and camera operators use light intensity knobs to control retina illumination. Bright white light flashes through the pupil to the back of the eye, and the imaging sensor produces a color image of the retina. This type of retina imaging device cannot achieve imaging of different layers of the retina, which causes certain obstacles to medical diagnosis.

The information disclosed in this background section is only intended to deepen the understanding of the overall background of the disclosure, and should not be taken as an acknowledgement or in any way suggesting that this information constitutes prior art that is well known to those skilled in the art.

### SUMMARY

The object of the present disclosure includes providing a retina digital imaging system to solve the technical problem that the retina imaging device in the prior art cannot achieve imaging of different layers of the retina and causes certain obstacles to medical diagnosis.

The object of the present disclosure includes providing a retina digital imaging instrument to improve the technical problem that the retina imaging device in the prior art cannot achieve imaging of different layers of the retina and causes certain obstacles to medical diagnosis.

The object of the present disclosure includes providing a retina digital imaging method to improve the technical problem that the retina imaging device in the prior art cannot achieve imaging of different layers of the retina and causes certain obstacles to medical diagnosis.

For one of the above objects, the present disclosure provides the following technical solutions:
The present disclosure provides a retina digital imaging system, including: an illumination module, a main optical assembly, and an image sensor module;
the illumination module includes light-emitting diodes configured to emit light with different wavelengths, and each of the light-emitting diodes is configured to emit light into a retina through the main optical assembly to form an illumination optical path; and
the light reflected by the retina is configured to pass through the main optical assembly to image on the image sensor module to form an imaging optical path.

As a further technical solution, the main optical assembly includes a ring reflector and an objective lens;
the ring reflector is configured to reflect the light emitted by the illumination module toward the objective lens to finally reach the retina;
the illumination optical path is configured to pass through the objective lens to the retina; and the imaging optical path is configured to pass through the objective lens to reach the image sensor module.

As a further technical solution, a non-reflective through channel is defined in a middle of the ring reflector, and the light reflected by the retina is configured to pass through the objective lens and then pass through the channel in the middle of the ring reflector to reach the image sensor module.

As a further technical solution, the objective lens includes lenses, and a surface of each of the lenses is configured to be coated with an anti-reflection coating.

As a further technical solution, the objective lens includes a basic front objective lens and an auxiliary lens sequentially spaced apart with the basic front objective lens.

As a further technical solution, the objective lens is configured such that a field of view formed by the retina digital imaging system in the retina is configured to be at least 55 degrees and at most 80 degrees.

As a further technical solution, the main optical assembly further includes a folded optical path reflector disposed in the imaging optical path and configured to fold the imaging optical path such that the light reflected by the retina is configured to reach the image sensor module; the folded optical path reflector is disposed between the ring reflector and the image sensor module in the imaging optical path.

As a further technical solution, the illumination optical path between the ring reflector and the illumination module and the imaging optical path between the folded optical path reflector and the image sensor module are configured to be parallel to each other.

As a further technical solution, the main optical assembly further includes a lateral compensator and an axial compensator, and the lateral compensator and the axial compensator are located at the imaging optical path. The lateral compensator is disposed between the image sensor module and the folded optical path reflector and configured to adjust a tolerance and alignment on a plane perpendicular to an optical axis of the imaging optical path, and the axial compensator is disposed between the image sensor module and the folded optical path reflector and configured to adjust an axial offset.

As a further technical solution, a diopter compensator is further provided between the folded optical path reflector and the image sensor module.

As a further technical solution, the retina digital imaging system further includes an imaging optical path field aperture disposed in the imaging optical path and between the diopter compensator and the image sensor module.

As a further technical solution, the retina digital imaging system further includes a field lens and an illumination optical path aperture which is configured to control a far-field illumination range; the field lens and the illumination optical path aperture are configured to be disposed in the illumination optical path, and the illumination optical path aperture is configured to be provided between the field lens and the illumination module.

As a further technical solution, the illumination module further includes a light energy monitoring module and an energy excess cut-off function module. The light energy monitoring module is configured to monitor and record energy of each of the light-emitting diodes each time the light-emitting diode emits light in real time, and the energy excess cut-off function module is configured to cut off a light source immediately when the energy of each of the light-emitting diodes emitting light once reaches a safety energy warning limit during normal or abnormal operation.

As a further technical solution, the retina digital imaging system further includes a sight target module and a beam splitter, and the sight target module is configured to transmit light to eyes through the beam splitter to guide a viewing direction of the eyes.

As a further technical solution, the sight target module and the image sensor module are configured to be on a same imaging plane.

As a further technical solution, the retina digital imaging system further includes an optical filter module providing separate filters for the illumination optical path and the imaging optical path; the optical filter module is configured to be disposed between the illumination module and the main optical assembly.

As a further technical solution, the optical filter module includes multiple pairs of filters, and is configured to simultaneously switch the filters used for the illumination optical path and the imaging optical path.

As a further technical solution, the retina digital imaging system further includes an illumination optical path lateral compensator, and the illumination optical path lateral compensator is configured to be disposed between the optical filter module and the ring reflector.

As a further technical solution, the image sensor module further includes an external trigger configured to synchronize a image shooting with an illumination flash.

The embodiments of the present disclosure further provide the following technical solutions:
The present disclosure provides a retina digital imaging instrument, including a central control module and the retina digital imaging system as described in any one of the above technical solutions. The central control module is configured to control and connect an illumination module and an image sensor module.

As a further technical solution, the retina digital imaging instrument further includes a real-time high-function embedded control software module operated on a computing platform with high capacity, high performance and high speed.

The embodiments of the present disclosure further provide the following technical solutions:
A retina digital imaging method, including:
emitting light with different wavelengths through light-emitting diodes, where each of the light-emitting diodes is configured to emit light into a retina through a main optical assembly to form an illumination optical path; and
the light reflected by the retina passing through the main optical assembly to image on an image sensor module to form an imaging optical path.

Compared with the prior art, the retina digital imaging system, retina digital imaging instrument, and retina digital imaging method provided by the present disclosure can achieve the following technical effects:
A retina digital imaging system provided by the present disclosure includes: an illumination module, a main optical assembly, and an image sensor module; the illumination module includes light-emitting diodes capable of emitting light with different wavelengths, and each light-emitting diode emits light to enter the retina through the main optical assembly to form an illumination optical path; the light reflected by the retina passes through the main optical assembly and forms an image on the image sensor module to form an imaging optical path. Multiple light-emitting diodes of the illumination module may emit light of different wavelengths, that is, different spectral bands may be provided. The reflection and absorption of the light by the retina depends on the spectrum, and the light with different wavelengths penetrates the retina at different depths. The various spectral bands formed by light-emitting diodes may form a wider spectrum, so different layers of the retina may be imaged to provide valuable medical and diagnostic data.

The present disclosure provides a retina digital imaging instrument, including a central control module and the retina digital imaging system as described in any one of the above technical solutions. The central control module is configured to control and connect the retina digital imaging system. The retina digital imaging instrument may obtain all the beneficial effects that the retina digital imaging system may achieve, and provides more convenient and reliable means and method for ophthalmological treatment and diagnosis.

A retina digital imaging method provided by the present disclosure may obtain all the beneficial effects that may be achieved by the above-mentioned retina digital imaging system, and may image different layers of the retina, thereby providing valuable medical and diagnostic data.

Other features and advantages of the present disclosure will be explained in the subsequent description, and partly become obvious from the description, or be understood by implementing the present disclosure. The objects and other advantages of the present disclosure are achieved and obtained by the structures specified in the description, claims, and drawings.

### BRIEF DESCRIPTION OF THE DRAWINGS

In order to more clearly explain the specific embodiments of the present disclosure or the technical solutions in the prior art, the following will briefly introduce the drawings required for the specific embodiments or the description of the prior art. Obviously, the drawings are some embodiments of the present disclosure. For those of ordinary skill in the art, without paying any creative labor, other drawings can also be obtained based on these drawings.
FIG. 1 is a working principle diagram of a retina digital imaging system provided in Embodiment one of the present disclosure;
FIG. 2 is a working principle diagram of the retina digital imaging system provided in another embodiment of the present disclosure; and
FIG. 3 is a schematic structural diagram of a retina digital imaging instrument provided in Embodiment two of the present disclosure.

Reference numerals: 1-illumination module; 2-image sensor module; 3-main optical assembly; 31-ring reflector; 32-objective lens; 33-folded optical path reflector; 34-lateral compensator; 35-axial compensator; 4 -diopter compensator; 5-sight target module; 51-beam splitter; 6-optical filter module; 7-alignment mechanism; 8-front alignment module; 9-display and user interface; 10-central control module; 11-field lens; 12-illumination optical path aperture; 13-illumination optical path lateral compensator.

### DETAILED DESCRIPTION OF THE EMBODIMENTS

The technical solutions of the present disclosure will be described clearly and completely below with reference to the drawings. Obviously, the described embodiments are part of the embodiments of the present disclosure, but not all of the embodiments. Based on the embodiments in the present disclosure, all other embodiments obtained by those of ordinary skill in the art without making creative efforts fall within the protection scope of the present disclosure.

In the description of the present disclosure, it should be noted that the terms "upper", "lower", "inner", "outer", etc. indicate the orientation or positional relationship based on the orientation or positional relationship shown in the drawings, only for the convenience of describing the present disclosure and simplify the description, rather than indicating or implying that the device or element referred to must have a specific orientation, be constructed and operate in a specific orientation, and therefore cannot be construed as limiting the present disclosure. In addition, the terms "first", "second", and "third" are for descriptive purposes only, and cannot be understood as indicating or implying relative importance.

In the description of the present disclosure, it should be noted that the terms "installation", "link", and "connection" should be understood in a broad sense, for example, it may be a fixed connection or a removable connection, or a integral connection; it may be either mechanical connection or electrical connection; it may be direct connection, or indirect connection through an intermediary, or internal connection between two components. For those of ordinary skill in the art, the specific meaning of the above terms in the present disclosure may be understood in specific situations.

The specific embodiments of the present disclosure will be described in detail below with reference to the drawings. It should be understood that the specific embodiments described herein are only used to illustrate and explain the present disclosure, and are not intended to limit the present disclosure.

### Embodiment one

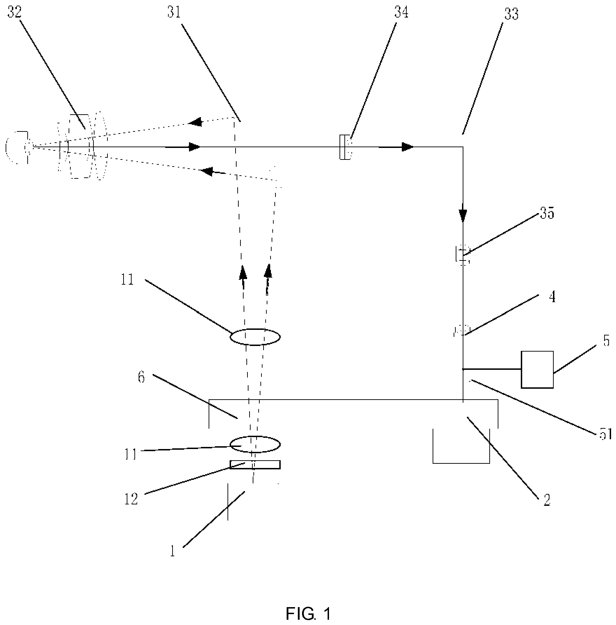

Referring to FIG. 1, an embodiment of the present disclosure provides a retina digital imaging system, including an illumination module 1, a main optical assembly 3, and an image sensor module 2.

The illumination module 1 includes light-emitting diodes capable of emitting light with different wavelengths, and each light-emitting diode emits light to enter the retina through the main optical assembly 3 to form an illumination optical path; the light reflected by the retina passes through the main optical assembly 3 and images on the image sensor module 2 to form an imaging optical path.

Further, the illumination module 1 may include one or more illumination units; when there are multiple illumination units, the multiple illumination units are arranged in parallel, and may emit light individually or simultaneously. Each illumination unit has a plurality of light-emitting diodes, and the light emitted by the plurality of light-emitting diodes has mutually different wavelengths. Certainly, in other embodiments of the present disclosure, the light-emitting elements used in the illumination unit may include different types of light-emitting diodes, for example, may include molecular light-emitting diodes, organic light-emitting diodes, or laser diodes, and the light-emitting elements may also include other laser sources other than laser diodes.

According to the retina digital imaging system provided by the embodiments of the present disclosure, the entire spectrum from blue to near infrared (wavelength 480nm to 980nm) is divided into different relatively narrow spectral bands. The light with various wavelengths is emitted through multiple light-emitting diodes respectively. Since the reflection and absorption of the light by the retina depends on the spectrum, different wavelengths may penetrate the retina at different depths, so different layers of the retina can be imaged, thereby obtaining valuable medical and diagnostic data.

It should be noted that the retina images irradiated by each light-emitting diode spectrum are collected separately.

The illumination optical path uniformly scatters the light output from the illumination module 1 through the pupil on the retina. The dedicated optical path for illumination ends at the ring reflector 31, at which point the beams of the illumination and imaging optical paths are combined coaxially. The entire illumination optical path has a magnification factor of 0.2-2.0, optionally, the magnification factor may be 0.5.

Please continue to refer to FIG. 1. In at least one embodiment of the present disclosure, the retina digital imaging system may further include a field lens 11 and an illumination optical path aperture 12. The illumination optical path aperture 12 is configured to control the far-field illumination range. The field lens 11 and the illumination optical path aperture 12 are provided in the illumination optical path. The illumination optical path aperture 12 is provided between the field lens 11 and the illumination module 1. The field lens 11 is provided between the illumination optical path aperture 12 and the main optical assembly 3. The light emitted by triggering is transmitted to an input end of the illumination optical path of the main optical assembly 3 through an optical coupling unit of the illumination module 1, and passes through the illumination optical path aperture 12, the illumination optical path eye lens and the field lens 11 to image the retina of the patient's eye, and then illuminate the entire retina within a predetermined solid angle (field of view). That is, the light emitted by the triggering of the illumination module 1 is transmitted to the input end of the illumination optical path of the main optical assembly 3 through the illumination optical path aperture 12 and the field lens 11 in sequence, so as to be imaged to the retina of the patient's eye.

The image sensor module 2 in this embodiment may be a Complementary Metal Oxide Semiconductor (CMOS), a Charge-coup Device (CCD), or other formats or technologies, which have the ability to record images continuously in a relatively high speed.

In addition, the image sensor module 2 in at least one embodiment of the present disclosure is further provided with an external trigger. The external trigger may be optionally an external fast electrical signal trigger interface, which may synchronize the image shooting with the illumination flash.

Further, the main optical assembly 3 in at least one embodiment of the present disclosure may include a ring reflector 31 and an objective lens 32; the ring reflector 31 is configured to reflect the light emitted by the illumination module 1 to the retina; the illumination optical path passes through the objective lens 32 to the retina; the imaging optical path passes through the objective lens 32 to reach the image sensor module 2 for information collection.

The light emitted by the illumination module 1 forms a ring-shaped light, which is irradiated on the ring reflector 31, and the ring reflector 31 reflects the light through the objective lens 32 to enter the retina.

Further, the included angle of the ring reflector 31 relative to the incident light emitted by the illumination module 1 may be 0-90o, further optionally be 45o, and the included angle of the ring reflector 31 relative to the reflected light reflected to the retina may also be 0-90o, further optionally be 45o.

It should be noted that the ring reflector 31 may be an integrated ring structure or a ring structure composed of multiple arc segments.

In addition, a non-reflective through channel is defined in the middle of the ring reflector 31 to facilitate the passage of the imaging optical path. That is, the light reflected by the retina passes through the objective lens 32 and then passes through the channel in the middle of the ring reflector 31 to reach the image sensor module 2.

Further, the illumination module 1 may further include a light energy monitoring module and an energy excess cut-off function module. The output optical power and energy of each light-emitting diode are calibrated. The light energy monitoring module is configured to monitor and record energy of each of the light-emitting diodes each time the light-emitting diode emits light in real time, and the energy excess cut-off function module is configured to cut off a light source immediately when the energy of each of the light-emitting diodes emitting light once reaches a safety energy warning limit during normal or abnormal operation. In this way, the illumination module 1 has functions of light energy monitoring and energy excess cut-off.

In at least one embodiment of the present disclosure, the objective lens 32 is composed of several lenses, which may include a basic front objective lens and an auxiliary lens arranged in sequence at intervals. The basic front objective lens is the key to imaging. In another embodiment of the present disclosure, the objective lens 32 may also include only the basic front objective lens and not include the auxiliary lens. In other words, the auxiliary lens may be optional, and may be one or more, and the auxiliary lens may be a convex lens or a concave lens. The function of the auxiliary lens is to improve image performance and resolution, and minimize aberrations. For example, the objective lens 32 may be composed of two lenses, a basic front objective lens, and a non-circular lens for compensating aberrations.

Further, the objective lens 32 realizes an increase in the field of view formed in the retina by the retina digital imaging system provided by the embodiments of the present disclosure. Optionally, the existing field of view of 40o is increased to 55-80o, and optionally may further be 63o, which increases the observation range, gets more comprehensive information of the retina, and provides more and more comprehensive medical reference data.

As a preferred embodiment, all lens surfaces of the objective lens 32 use anti-reflective coating technology to reduce unnecessary reflections.

It should be noted that all the lens surfaces in the retina digital imaging system provided in at least one embodiment of the present disclosure adopt the above-mentioned anti-reflection coating technology. In other words, all lens surfaces of the objective lens 32 may be provided with the anti-reflection coating.

The main optical assembly 3 in at least one embodiment of the present disclosure may further include a folded optical path reflector 33 disposed in the imaging optical path and configured to reflect light reflected by the retina to the image sensor module 2.

In this embodiment, the image sensor module 2 and the retina are not on the same straight line. In order to smoothly pass the light reflected by the retina to the image sensor module 2, a folded optical path reflector 33 is provided on the path of the imaging optical path. The folded optical path reflector 33 is provided between the ring reflector 31 and the image sensor module 2 in the path of the imaging optical path. The light reflected from the retina reaches the image sensor module 2 through the objective lens 32 and the folded optical path reflector 33. During this process, the light folds, thus reducing the size of the imaging instrument.

It should be noted that in such a structural design, the illumination optical path between the ring reflector 31 and the illumination module 1 and the imaging optical path between the folded optical path reflector 33 and the image sensor module 2 are parallel to each other, thus the entire structure is more compact, and the operation is also more convenient.

In at least one embodiment of the present disclosure, the main optical assembly 3 further includes a lateral compensator 34 and an axial compensator 35, and the lateral compensator 34 and the axial compensator 35 are located on the imaging optical path. The lateral compensator 34 is configured to adjust a tolerance and alignment on a plane perpendicular to an optical axis of the imaging optical path. The axial compensator 35 is configured to adjust an axial offset. The lateral compensator 34 is disposed between the ring reflector 31 and the folded optical path reflector 33, which is realized by a lens with a lateral position adjustable. The axial compensator 35 is disposed between the folded optical path reflector 33 and the image sensor module 2, which is realized by a lens with an axial position adjustable.

Further, a diopter compensator 4 is further provided between the folded optical path reflector 33 and the image sensor module 2. The diopter compensator 4 is a part of the imaging system. Diopter compensation is achieved by adjusting the axial position of a lens and then changing the focal length of the imaging system.

Further, the folded optical path reflector 33, the axial compensator 35, the diopter compensator 4 and the image sensor module 2 may be arranged sequentially.

The above-mentioned diopter compensator 4 is dynamic or adjustable, and may achieve focus control, which is achieved specifically through a quasi-telecentric lens group, so that the imaging optical path is within the entire +15 to -15 diopter range, and the image size changes within only ± 10% .

Further, the retina digital imaging system may further includes an imaging optical path field aperture disposed in the imaging optical path and between the diopter compensator 4 and the image sensor module 2.

The retina imaging system in at least one embodiment of the present disclosure may further include a sight target module 5 configured to guide the observation direction of the eye.

The sight target module 5 and the image sensor module 2 are configured to be on a same imaging plane. The retina imaging system in at least one embodiment of the present disclosure may further include a beam splitter 51 disposed between the folded optical path reflector 33 and the image sensor module 2. The beam splitter 51 guides the light toward the sight target module 5 to transmit the light to the eye to guide the observation direction of the eye. However, the sight target module 5 and the image sensor module 2 are separately installed and arranged by separating the optical paths through the beam combiner. The position of the gaze target guides the eyes to look in different directions, thus allowing imaging of different parts of the retina. The function of displaying position of the sight target module 5 may be implemented by different methods, such as a light-emitting diode array, being based on a liquid crystal display, or a dynamic micromirror matrix. Both the liquid crystal display and the dynamic micromirror matrix have high-resolution and fully programmable image generators.

The retina imaging system in at least one embodiment of the present disclosure may further include an optical filter module 6 providing separate filters for the illumination optical path and the imaging optical path. Optionally, the optical filter module 6 includes multiple pairs of filters, and may simultaneously switch the filters used for the illumination optical path and the imaging optical path.

The filter module configured for self-fluorescence imaging and other functions will provide separate filters for the illumination light beam and the observation light beam. The two channels of filters are carried at corresponding positions on the same filter disc.

The filter disc is controlled and rotated by the control motor. For the illumination optical path and the imaging optical path, when the filter disc is driven and rotated by the control motor, the filter configured for the illumination optical path to pass through and the filter configured for the imaging optical path to pass through are always switched with the exact lock step.

It should be understood that in at least one embodiment of the present disclosure, there may be two field lenses 11. The illumination module 1, the illumination optical path aperture 12, one field lens 11, the optical filter module 6, the other field lens 11, the ring reflector 31 and the objective lens 32 are sequentially arranged. The light emitted by the illumination module 1 passes through the illumination optical path aperture 12, one field lens 11, the optical filter module 6, the other field lens 11, the ring reflector 31, and the objective lens 32 in sequence to enter the retina to form the illumination optical path. In addition, the objective lens 32, the ring reflector 31, the lateral compensator 34, the folded optical path reflector 33, the axial compensator 35, the diopter compensator 4, the beam splitter 51 and the image sensor module 2 are sequentially arranged. The light reflected by the retina passes through the objective lens 32, the ring reflector 31, the lateral compensator 34, the folded optical path reflector 33, the axial compensator 35, the diopter compensator 4 and the beam splitter in sequence, and images on the image sensor module 2 to form the imaging optical path. The beam splitter 51 guides the light toward the sight target module 5 to transmit the light to the eye to guide the observation direction of the eye.

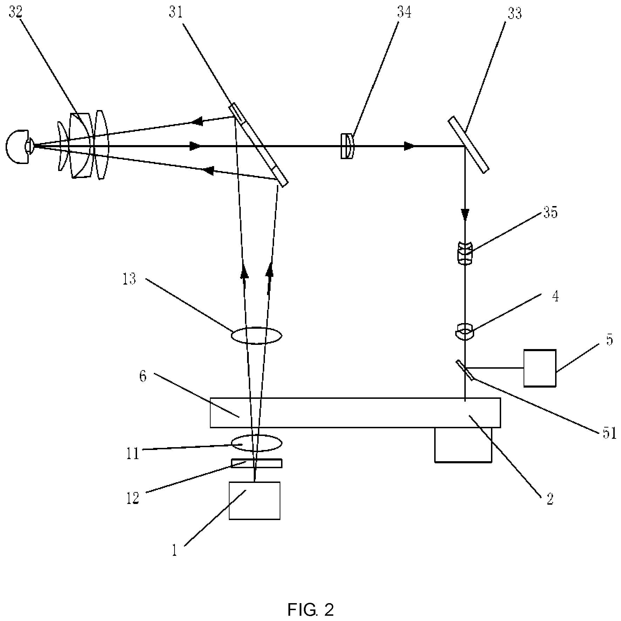

Please refer to FIG. 2. It should be understood that in at least one embodiment of the present disclosure, the retina digital imaging system may include an illumination optical path lateral compensator 13, which may replace the field lens 11 between the optical filter module 6 and the ring reflector 31 in the above embodiment. That is, the illumination module 1, the illumination optical path aperture 12, the field lens 11, the optical filter module 6, the illumination optical path lateral compensator 13, the ring reflector 31 and the objective lens 32 are sequentially arranged, and the light emitted by the illumination module 1 passes through the illumination optical path aperture 12, the field lens 11, the optical filter module 6, the illumination optical path lateral compensator 13, the ring reflector 31 and the objective lens 32 in sequence to enter the retina to form the illumination optical path. The illumination optical path lateral compensator 13 is configured to adjust a tolerance and alignment on a plane perpendicular to an optical axis of the illumination optical path.

In summary, the retina digital imaging system provided by the present disclosure includes: an illumination module 1, a main optical assembly 3, and an image sensor module 2; the illumination module 1 includes light-emitting diodes capable of emitting light with different wavelengths, and each light-emitting diode emits light to enter the retina through the main optical assembly 3 to form an illumination optical path; the light reflected by the retina passes through the main optical assembly 3 and forms an image on the image sensor module 2 to form an imaging optical path. Multiple light-emitting diodes of the illumination module 1 may emit light of different wavelengths, that is, different spectral bands may be provided. The reflection and absorption of the light by the retina depends on the spectrum, and the light with different wavelengths penetrates the retina at different depths. The various spectral bands formed by light-emitting diodes may form a wider spectrum, so different layers of the retina may be imaged to provide valuable medical and diagnostic data.

### Embodiment two

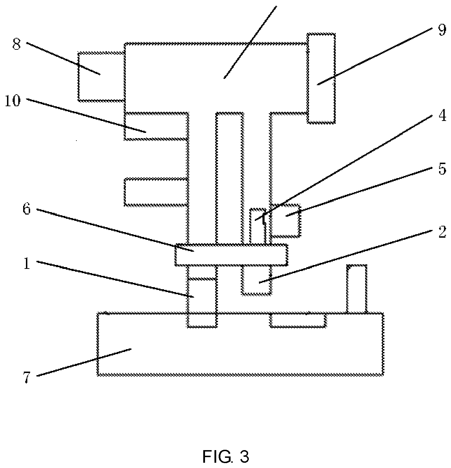

Referring to FIG. 3, an embodiment of the present disclosure provides a retina digital imaging instrument, including a central control module 10 and any of the retina digital imaging systems provided in the embodiment one above. The central control module 10 is configured to control and connect an illumination module 1 and an image sensor module 2.

The specific structure of the retina digital imaging system has been described in detail above, and will not be repeated here.

The entire retina digital imaging instrument structure further includes an alignment mechanism 7, a front alignment module 8, a display (not shown) and a user interface 9.

The central control module 10 is configured to coordinate and control the operation of the entire device (including the illumination module 1, the image sensor module 2, the alignment mechanism 7, the front alignment module 8, and the sight target module 5). The central control module 10 manages simultaneously all images transmitted to the display and user interface 9, and accept and coordinate the execution of all instructions input by the operator through the display and user interface 9. The control management, instruction execution and coordination of the central control module 10 are implemented through the control plane and control logic.

The central control module 10 includes a real-time high-function embedded control software module operated on a computing platform with high-capacity, high-performance and high-speed.

The central control module 10 includes a main controller and an internal memory, wherein the main controller controls the startup and shutdown of a plurality of light-emitting diodes, and at the same time, the main controller controls the operation of the image sensor module 2 and stores the information collected by the image sensor module 2 to the internal memory.

Another embodiment of the present disclosure further provides a retina digital imaging method, which can apply the retina digital imaging system and the retina digital imaging instrument provided in any of the above embodiments. The specific structures of the retina digital imaging system and the retina digital imaging instrument have been described in detail above, and will not be repeated here. The retina digital imaging method includes:
emitting light with different wavelengths through light-emitting diodes, where each of the light-emitting diodes is configured to emit light into a retina through a main optical assembly to form an illumination optical path, wherein, a plurality of light-emitting diodes form the illumination module 1; and
the light reflected by the retina passing through the main optical assembly 3 to image on an image sensor module 2 to form an imaging optical path.

Further, light with different wavelengths are emitted through a plurality of light-emitting diodes, and the light sequentially passes through the illumination optical path aperture 12, the field lens 11, the optical filter module 6, the illumination optical path lateral compensator 13, the ring reflector 31, and the objective lens 32 to enter the retina to form the illumination optical path. In addition, the light reflected by the retina passes through the objective lens 32, the ring reflector 31, the lateral compensator 34, the folded optical path reflector 33, the axial compensator 35, the diopter compensator 4 and the beam splitter in sequence, and images on the image sensor module 2 to form the imaging optical path.

Finally, it should be noted that the above embodiments are only used to illustrate the technical solutions of the present disclosure, but not to limit them. Although the present disclosure has been described in detail with reference to the foregoing embodiments, those of ordinary skill in the art should understand that: the technical solutions described in the foregoing embodiments may still be modified, or some or all of the technical features thereof may be equivalently replaced; and these modifications or replacements do not deviate the essence of the corresponding technical solutions from the scope of the embodiments of the present disclosure.

### INDUSTRIAL APPLICABILITY

The retina digital imaging system, retina digital imaging instrument, and retina digital imaging method provided by the embodiments of the present disclosure may image different layers of the retina, thereby providing valuable medical and diagnostic data

## Claims

1. A retina digital imaging system, **characterized by** comprising: an illumination module, a main optical assembly and an image sensor module;
wherein, the illumination module comprises light-emitting diodes configured to emit light with different wavelengths, and each of the light-emitting diodes is configured to emit light into a retina through the main optical assembly to form an illumination optical path; and
the light reflected by the retina is configured to pass through the main optical assembly to image on the image sensor module to form an imaging optical path.

2. The retina digital imaging system according to claim 1, **characterized in that** the main optical assembly comprises a ring reflector and an objective lens;
the ring reflector is configured to reflect the light emitted by the illumination module toward the objective lens to finally reach the retina;
the illumination optical path is configured to pass through the objective lens to the retina; and
the imaging optical path is configured to pass through the objective lens to reach the image sensor module.

3. The retina digital imaging system according to claim 2, **characterized in that**: a non-reflective through channel is configured to be defined in a middle of the ring reflector, and the light reflected by the retina is configured to pass through the objective lens and then pass through the channel in the middle of the ring reflector to reach the image sensor module.

4. The retina digital imaging system according to claim 2 or 3, **characterized in that** the objective lens comprises lenses, and a surface of each of the lenses is configured to be coated with an anti-reflection coating.

5. The retina digital imaging system according to any one of claims 2 to 4, **characterized in that** the objective lens comprises a basic front objective lens and an auxiliary lens sequentially spaced apart with the basic front objective lens.

6. The retina digital imaging system according to any one of claims 2 to 5, **characterized in that** the objective lens is configured such that a field of view formed by the retina digital imaging system in the retina is configured to be at least 55 degrees and at most 80 degrees.

7. The retina digital imaging system according to any one of claims 2 to 6, **characterized in that** the main optical assembly further comprises a folded optical path reflector disposed in the imaging optical path and configured to fold the imaging optical path such that the light reflected by the retina is configured to reach the image sensor module; the folded optical path reflector is disposed between the ring reflector and the image sensor module in the imaging optical path.

8. The retina digital imaging system according to claim 7, **characterized in that** the illumination optical path between the ring reflector and the illumination module and the imaging optical path between the folded optical path reflector and the image sensor module are configured to be parallel to each other.

9. The retina digital imaging system according to claim 7 or 8, **characterized in that** the main optical assembly further comprises a lateral compensator and an axial compensator, the lateral compensator and the axial compensator being located at the imaging optical path, the lateral compensator being disposed between the image sensor module and the folded optical path reflector and configured to adjust a tolerance and alignment on a plane perpendicular to an optical axis of the imaging optical path, the axial compensator being disposed between the image sensor module and the folded optical path reflector and configured to adjust an axial offset.

10. The retina digital imaging system according to claim 9, **characterized in that** a diopter compensator is further provided between the folded optical path reflector and the image sensor module.

11. The retina digital imaging system according to claim 10, **characterized by** further comprising an imaging optical path field aperture disposed in the imaging optical path and between the diopter compensator and the image sensor module.

12. The retina digital imaging system according to any one of claims 1 to 11, **characterized by** further comprising a field lens and an illumination optical path aperture configured to control a far-field illumination range; the field lens and the illumination optical path aperture are configured to be disposed between the main optical assembly and the illumination module, and the illumination optical path aperture is configured to be provided between the field lens and the illumination module.

13. The retina digital imaging system according to any one of claims 1 to 12, **characterized in that** the illumination module further comprises a light energy monitoring module and an energy excess cut-off function module, the light energy monitoring module being configured to monitor and record energy of each of the light-emitting diodes each time the light-emitting diode emits light in real time, the energy excess cut-off function module being configured to cut off a light source immediately when the energy of each of the light-emitting diodes emitting light once reaches a safety energy warning limit during normal or abnormal operation.

14. The retina digital imaging system according to any one of claims 1 to 13, **characterized by** further comprising a sight mark module and a beam splitter, and the sight mark module is configured to transmit light to eyes through the beam splitter to guide a viewing direction of the eyes.

15. The retina digital imaging system according to any one of claims 1 to 14, **characterized in that** the sight mark module and the image sensor module are configured to be on a same imaging plane.

16. The retina digital imaging system according to any one of claims 1 to 15, **characterized by** further comprising an optical filter module providing separate filters for the illumination optical path and the imaging optical path.

17. The retina digital imaging system according to any one of claims 1 to 16, **characterized in that** the image sensor module further comprises an external trigger configured to synchronize a image shooting with an illumination flash.

18. A retina digital imaging instrument, **characterized by** comprising a central control module and the retina digital imaging system as recited in any one of claims 1 to 17, and the central control module is configured to control and connect an illumination module and an image sensor module.

19. The retina digital imaging instrument according to claim 18, **characterized by** further comprising a real-time high-function embedded control software module operated on a computing platform with high capacity, high performance and high speed.

20. A retina digital imaging method, **characterized by** comprising:
emitting light with different wavelengths through light-emitting diodes, wherein each of the light-emitting diodes emits light into a retina through a main optical assembly to form an illumination optical path; and
the light reflected by the retina passing through the main optical assembly to image on an image sensor module to form an imaging optical path.
